# EUROPÄISCHE PATENTSCHRIFT

(11) **EP 1 358 106 B1**
(45) Veröffentlichungstag und Bekanntmachung des Hinweises auf die Patenterteilung: **02.06.2004**
(21) Anmeldenummer: 02779094.8
(22) Anmeldetag: 02.12.2002
(51) Int. Cl.: B64D 10/00

(54) **VORRICHTUNG ZUM MESSEN DER ATEMFREQUENZ**
DEVICE FOR MEASURING THE RESPIRATORY RATE
DISPOSITIF DE MESURE DE LA FREQUENCE RESPIRATOIRE

(30) Priorität: 20.12.2001 CH 232401
(43) Veröffentlichungstag der Anmeldung: 05.11.2003
(73) Patentinhaber: Prospective Concepts AG, 8152 Glattbrugg (CH)
(72) Erfinder: EGLI, Wendelin, CH-8472 Seuzach (CH)
(74) Vertreter: Salgo, Reinhold Caspar, Dr.
(86) Internationale Anmeldenummer: PCT/CH2002/000648
(87) Internationale Veröffentlichungsnummer: WO 2003/053780

(56) Entgegenhaltungen:
- EP-A- 0 376 027
- EP-A- 0 498 079
- EP-B- 0 986 356
- WO-A-86/00793

## Beschreibung

Die vorliegende Erfindung bezieht sich auf eine Vorrichtung zum Messen der Atemfrequenz und des Atemmusters beispielsweise des Trägers eines Beschleunigungs-Schutzanzuges auf dem hydrostatischen Prinzips nach dem Oberbegriff des Patentanspruches 1.

Vorrichtungen zum Ermitteln der physiologischen Daten von Piloten, Sporttreibenden oder beispielsweise Orthostase-Patienten, wie Puls, Blutsauerstoffgehalt, Atemfrequenz, sind mehrere bekannt. Im Allgemeinen sind es Weiterentwicklungen oder besondere Ausführungen von Messgeräten, wie sie in der Medizin, insbesondere der Sportmedizin verwendet werden.

Ein nahezu gemeinsames Merkmal solcher Messvorrichtungen ist es, dass ein geeigneter Sensor an der Probandin, am Probanden angebracht werden muss und ein bestimmtes Mass von Störung, wenn nicht Beeinträchtigung mindestens des subjektiven Wohlbefindens mit sich bringen. Damit besteht die Gefahr der Minderung der Akzeptanz solcher Messvorrichtungen, wenn nicht gar der Entstehung von Artefakten: Durch die Existenz der Messvorrichtung bewirkte Fehlfunktionen der Probanden.

Die Aufgabe der vorliegenden Erfindung ist die Schaffung einer genannten Vorrichtung zur Messung der Atemfrequenz, welche mit einem Minimum von Eingriffen in die übliche Umgebung des Probanden auskommt, kostengünstig hergestellt und eingebaut bzw. angebracht werden kann und unter schwierigen physikalischen und physiologischen Verhältnissen zuverlässige Resultate liefert.

Die Lösung der gestellten Aufgabe ist wiedergegeben im kennzeichnenden Teil des Patentanspruches 1 hinsichtlich ihrer wesentlichen Merkmale, in den folgenden Ansprüchen hinsichtlich weiterer vorteilhafter Ausbildungen.

Anhand der beigefügten Zeichnung wird die Erfindung näher erläutert. Es zeigen
Fig. 1a in schematischer Weise die erfindungsgemässe Vorrichtung, Fig. 1b die Anordnung von Fig. 1a im Querschnitt,
Fig. 2 ein Blockschaltbild,
Fig. 3 ein erstes Druck-Zeitdiagramm,
Fig. 4 ein zweites Druck-Zeitdiagramm.

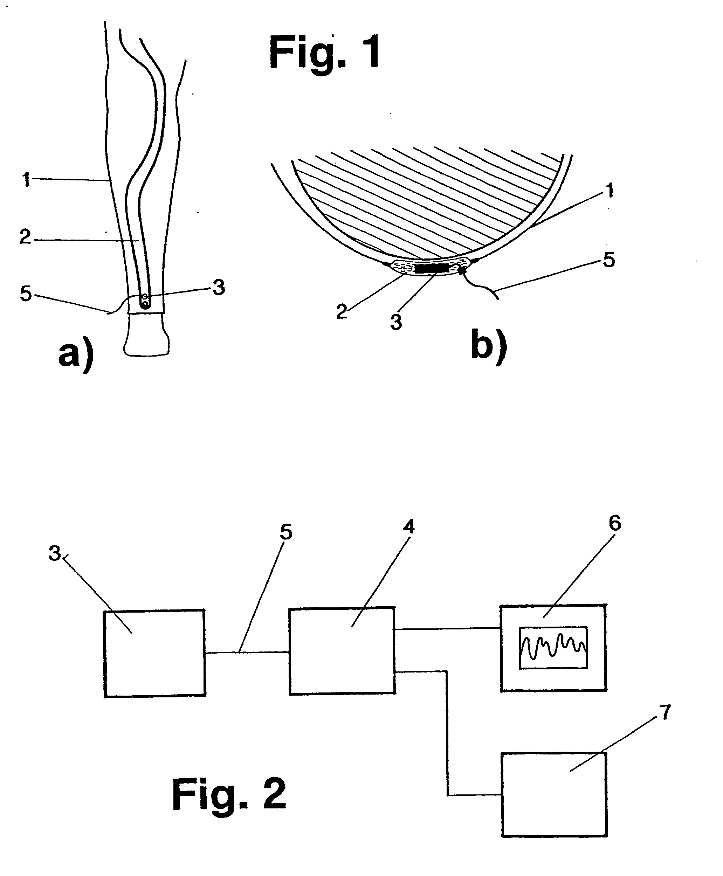

Fig. 1a, b sind schematische Darstellungen der erfindungsgemässen Anordnung zum Einsatz in einem Beschleunigungs-Schutzanzug, einem Orthostase-Anzug oder einem sog. Hypoxi-Kleidungsstück; Fig. 1a in einer Draufsicht von vorne, Fig. 1b in einem Querschnitt. Ein nach dem hydrostatischen Prinzip arbeitender Beschleunigungs-Schutzanzug 1 (fortan Anzug genarmt, beispielsweise nach EP 0 983 190 oder EP 0 986 356, weist flüssigkeitsnannt, gefüllte Adern 2 auf, welche in den Anzug 1 eingearbeitet sind, verlaufend in Längsrichtung der Glieder des Trägers dieses Anzuges 1. Beispielsweise im tiefstmöglichen Punkt einer der Adern 2, in der Regel oberhalb des Fusses, ist eine Druckmesszelle 3 eingefügt, dergestalt, dass sie völlig von der die Ader 2 füllenden Flüssigkeit umgeben ist. In geeigneter Weise ist die Druckmesszelle 3 an ein mehradriges Kabel 5 mit einem in Fig. 2 dargestellten Auswertegerät 4 verbunden. Das Kabel 5 kann in die Ader 2 entweder durch eine druckdichte Durchführung eingeführt oder an einen druckdicht angebrachten Stecker angeschossen werden. Innerhalb des Erfindungsgedankens ist auch eine Signalübertragung aus der Ader nach aussen mittels Optokopplers oder über Funk, Wie allgemein üblich bei Telemetrieaufgaben und insbesondere bei solchen in der Biomechanik. Die Druckmesszelle 3 ist an sich bekannt und beispielsweise vom selbstkalibrierenden Typ. Ferner ist auch durchaus möglich, an die Ader 2, beispielsweise mittels eines Schlauches, ein die Druckmesszelle 3 enthaltendes Gefäss anzuschliessen, wobei die Druckmesszelle 3 in beschriebener Art mit dem Kabel 5 verbunden ist. Die Druckmesszelle 3 steht also in flüssigkeits- und druckkommunizierender Verbindung mit einer der Adern 2. Fig. 2 zeigt das Blockschema der erfindungsgemässen Vorrichtung.

Mittels des Kabels 5 ist die Druckmesszelle 3 mit dem Auswertegerät 4 verbunden. Dieses bereitet die Druckmesswerte in digitaler Form auf, unter Berücksichtigung der Eichwerte der. Druckmesszelle 3. Diese aufbereiteten Messwerte können entweder auf einer Anzeigevorrichtung 6 direkt im Zeitablauf visualisiert, oder aber einer Speichereinrichtung 7 zur Speicherung zugeleitet werden. Eine solche Speichereinrichtung kann die Speicherung weiterer persönlicher Parameter wie beispielsweise Puls, Oxymetriedaten, EKG, EOG eingerichtet sein.

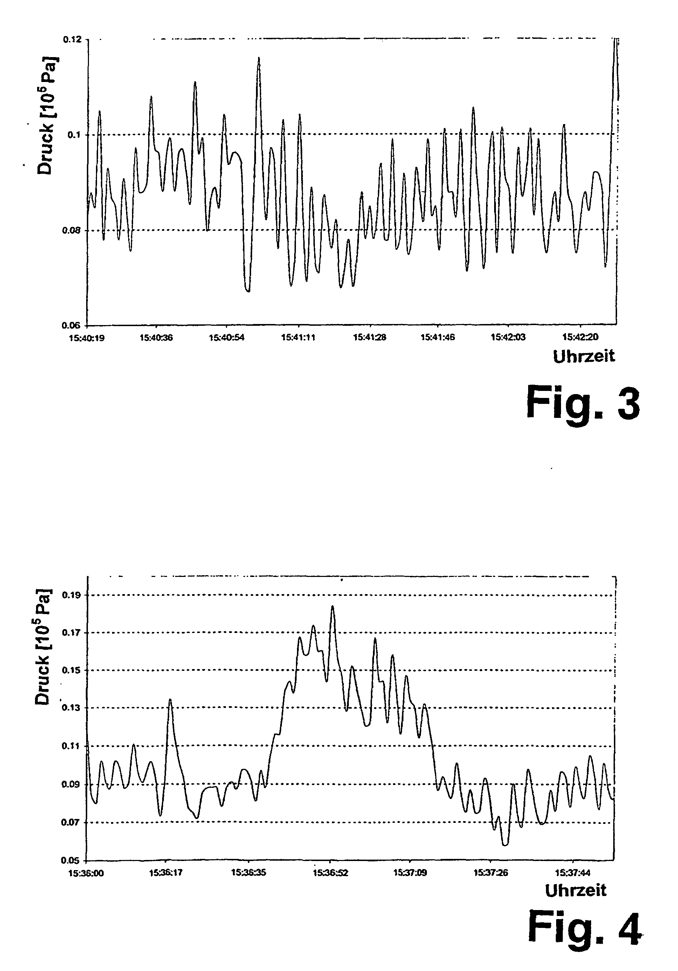

Bei der Verwendung eines genannten Anzuges 1, ist es wesentlich, dass sein Sitz vor dem Flug überprüft wird. Da das Grundmaterial des Anzuges aus wenig dehnbarem Gewebe, beispielsweise aus Aramid-Fasern, besteht, ist die Qualität des Sitzes von den momentanen körperlichen Bedingungen des Trägers oder der Trägerin des Anzuges 1 abhängig. Nur wenn der Sitz genügend straff eingestellt ist, kann der Anzug 1 seine Aufgabe, nämlich des Verhindern des Blutabstromes in die Abdominal-Region und die unteren Extremitäten, richtig erfüllen. Bei richtig eingestelltem Sitz ergibt sich ein Druck-Diagramm gemäss Fig. 3. Hier ist ein Druck-Zeitdiagramm dargestellt, aufgenommen mit der erfindungsgemässen Vorrichtung während des Geradeausfluges eines Kampfflugzeuges.

Überlagert über einem statischen Druck von etwa 90 hPa erscheint ein pulsierendes Druckmuster, welches die Atmung des Piloten abbildet. Die Atemfrequenz ist anhand des Zeitmessstabes Stunden/Minuten/Sekunden leicht feststellbar und beträgt hier etwa 24 Atemzüge/Minute. Das Atemdruckbild ist überlagert durch leichte Bewegungen sowohl des Piloten als auch des Flugzeuges. Ersteres zeigt sich in raschen, letzteres in langsameren Verschiebungen des Oszillations-Nullpunktes des Atemdruckes.

Da das Volumen des Anzuges nur sehr wenig variabel ist, bewirkt das Einatmen eine leichte Volumenzunahme des Piloten, was sich in einem Anstieg der hydrostatischen Flüssigkeitssäule und damit des Binnendruckes des Anzuges äussert.

Fig. 4 ist ein Druck-Zeit-Diagramm, aufgenommen während eines Flugmanövers mit erhöhter lokaler z-Beschleunigung während etwa 40 sec. Auch hier ist die durch das Atmen bewirkte Druckvariation deutlich sichtbar. Mit an sich bekannten Methoden der Datenverarbeitung lassen sich solche Druck-Zeit-Funktionen beispielsweise aufbereiten und in die einzelnen hier überlagerten Funktionen wie z-Beschleunigung und Puls trennen und einzeln beurteilen.

Insbesondere lassen sich Aspekte wie richtiger Sitz, Atemtechnik des Piloten und allenfalls mehr flugtechnische Parameter einzeln und detailliert beurteilen. Ferner ist es für den Piloten selbst wichtig, den richtigen Sitz - beispielsweise auf Grund der Druckamplitude - vor dem Start objektiv beurteilen zu können, was durch Betrachten des Bildes auf der Anzeigevorrichtung vorgesehen und möglich ist.

Beim Fliegen von Hochleistungsflugzeugen mit ihren Möglichkeiten enge Kurvenradien bei hohen Geschwindigkeiten zu ertragen, ist es entscheidend, dass der Pilot eine dementsprechende Atemtechnik beherrscht. Diese Atemtechnik ist flugmedizinisch indiziert und erlernbar. Als Lernquittung dient die Darstellung des Atemverlaufes auf der Anzeigevorrichtung 6. Selbstverständlich kann die Druckmesszelle 3 auch an einer anderen Stelle des Anzuges in einer Flüssigkeit führenden Ader 2 angebracht werden, so beispielsweise in der Thorax-Region.

Wird die Druckmesszelle 3 jedoch, wie eingangs beschrieben, an der tiefstmöglichen Stelle einer Ader 2 eingesetzt, so kann sie gleichzeitig als Messeinrichtung für die lokale z-Beschleunigung dienen. Ferner ist dann das Atemmuster deutlich vom beschleunigungsinduzierten Druck abgesetzt, wie in Fig. 4 sichtbar ist.

Selbstverständlich ist der Einsatz der erfindungsgemässen Vorrichtung in einem Orthostase-Anzug, beispielsweise gemäss EP 0 986 356, oder einem sog. Hypoxi-Kleidungsstück, beispielsweise gemäss der GH-Patentanmeldung 1610/02, ebenfalls möglich und aus medizinischen Gründen allenfalls angezeigt.

Bei einem genannten Hypoxi-Kleidungsstück wird die Atemfrequenz-Messeinrichtung - mangels flüssigkeitsführender Adern - in einer flüssigkeitsgefüllten Tasche einfach unter die elastisch vorgespannte Haut des Kleidungsstückes geschoben und dort angemessen befestigt.

## Patentansprüche

1. Vorrichtung zum Messen der Atemfrequenz und des Atemmusters des Trägers eines Beschleunigungsschutzanzuges auf dem hydrostatischen Prinzip mit flüssigkeitsgefüllten Adern (2), welche sich im Wesentlichen über die ganze Länge des Beschleunigungsschutzanzuges eines Orthostase-Anzuges oder eines HypoxiKleidungsstückes erstecken können, **dadurch gekennzeichnet, dass**
- eine Druckmesszelle (3) vorhanden ist, welche in einer flüssigkeitsgefüllten, flüssigkeitsdichten Hülle und druckkommunizierenden Verbindung steht mit einer der Adern (2),
- ein Auswertegerät (4) vorhanden ist, welches die Messwerte der Druckmesszelle (3) auswertet und aufbereitet und so eingerichtet ist, das es sowohl eine Anzeigevorrichtung (6), als auch eine Speichereinrichtung (7) speisen kann.

2. Vorrichtung nach Patentanspruch 1, **dadurch gekennzeichnet, dass** die Druckmesszelle (3) im Inneren einer Ader (2) eines Beschleunigungs-Schutzanzuges angebracht ist.

3. Vorrichtung nach Patentanspruch 1, **dadurch gekennzeichnet, dass** die Druckmesszelle (3) im Inneren eines Gefässes angebracht ist, welches mittels eines Schlauches an eine Ader (2) des Beschleunigungs-Schutzanzuges so angeschlossen ist, dass es mit dieser Ader (2) in flüssigkeits- und druckkommunizierender Weise in Verbindung steht.

4. Vorrichtung nach Patentanspruch 2 oder 3, **dadurch gekennzeichnet, dass** die Druckmesszelle (3) sich auf der Höhe der tiefsten Stelle der Ader (2) des Beschleunigungs-Schutzanzuges befindet.

5. Vorrichtung nach Patentanspruch 2 oder 3, **dadurch gekennzeichnet, dass** die Druckmesszelle (3) mittels eines Kabels (5) mit dem Auswertegerät (4) verbunden ist, welches Kabel (5) die von der Druckmesszelle (3) ermittelten Werte an das Auswertegerät (4) überträgt.

6. Vorrichtung nach Patentanspruch 2 oder 3, **dadurch gekennzeichnet, dass** ein Optokoppler vorhanden und an die Druckmesszelle (3) angeschlossen ist, welcher die von der Druckmesszelle (3) ermittelten Werte an das Auswertegerät (4) überträgt.

7. Vorrichtung nach Patentanspruch 2 oder 3, **dadurch gekennzeichnet, dass** ein Funkgerät vorhanden und an die Druckmesszelle (3) angeschlossen ist, welcher die von der Druckmesszelle (3) ermittelten Werte an das Auswertegerät (4) überträgt.

8. Vorrichtung nach Patentanspruch 1, **dadurch gekennzeichnet, dass** eine Anzeigevorrichtung (6) vorhanden und an das Auswertegerät (4) angeschlossen ist.

9. Vorrichtung nach Patentanspruch 1, **dadurch gekennzeichnet, dass** eine Speichervorrichtung (7) vorhanden und an das Auswertegrät (4) angeschlossen ist.

10. Vorrichtung nach Patentanspruch 1, **dadurch gekennzeichnet, dass** sowohl eine Anzeigevorrichtung (6) als auch eine Speichervorrichtung (7) vorhanden ist, und beide an das Auswertegerät (4) angeschlossen sind.

## Claims

1. A device for measuring the respiration rate and the breathing pattern of a person wearing an anti-blackout suit operating according to the hydrostatic principle, with liquid-conveying veins (2) which can extend substantially the entire length of the anti-blackout suit, an orthostasis suit or a hypoxia garment, **characterized in that**
- a pressure measurement cell (3) is present which is inside a liquid-filled, liquid-tight sheath and is in pressure-communicating connection with one of the veins (2),
- an evaluation apparatus (4) is present which evaluates and processes the measurement values of the pressure measurement cell (3) and is set up in such a way that it can feed both a display device (6) and a memory device (7).

2. The device as claimed in patent claim 1, **characterized in that** the pressure measurement cell (3) is arranged in the inside of a vein (2) of an anti-blackout suit.

3. The device as claimed in patent claim 1, **characterized in that** the pressure measurement cell (3) is arranged in the inside of a vessel which is connected via a tube to a vein (2) of the anti-blackout suit in such a way that it communicates with this vein (2) in a liquid-communicating and pressure-communicating manner.

4. The device as claimed in patent claim 2 or 3, **characterized in that** the pressure measurement cell (3) is situated at the level of the lowest point of the vein (2) of the anti-blackout suit.

5. The device as claimed in patent claim 2 or 3, **characterized in that** the pressure measurement cell (3) is connected via a cable (5) to the evaluation apparatus (4), which cable (5) transmits to the evaluation apparatus (4) the values determined by the pressure measurement cell (3).

6. The device as claimed in patent claim 2 or 3, **characterized in that** an optocoupler is present and is linked to the pressure measurement cell (3), said optocoupler transmitting to the evaluation apparatus (4) the values determined by the pressure measurement cell (3).

7. The device as claimed in patent claim 2 or 3, **characterized in that** a radio apparatus is present and is linked to the pressure measurement cell (3), said radio apparatus transmitting to the evaluation apparatus (4) the values determined by the pressure measurement cell (3).

8. The device as claimed in patent claim 1, **characterized in that** a display device (6) is present and is linked to the evaluation apparatus (4).

9. The device as claimed in patent claim 1, **characterized in that** a memory device (7) is present and is linked to the evaluation apparatus (4).

10. The device as claimed in patent claim 1, **characterized in that** both a display device (6) and also a memory device (7) are present and both are linked to the evaluation apparatus (4).

## Revendications

1. Dispositif de mesure de la fréquence respiratoire et du modèle respiratoire du porteur d'une combinaison de protection contre l'accélération sur la base du principe hydrostatique avec des veines (2) emplies de liquide qui peuvent s'étendre substantiellement sur toute la longueur de la combinaison de protection contre l'accélération, d'une combinaison d'orthostase ou d'une pièce d'habillement hypoxie, **caractérisé en ce que**
- une cellule de mesure de pression (3) est prévue, laquelle se trouve dans une enveloppe emplie de liquide, étanche aux liquides et en communication par pression avec une des veines (2),
- un appareil d'évaluation (4) est prévu, lequel évalue et édite les valeurs de mesure de la cellule de mesure de pression (3) et est prévu pour pouvoir alimenter tant un dispositif de visualisation (6) qu'un dispositif à mémoire (7).

2. Dispositif selon la revendication 1, **caractérisé en ce que** la cellule de mesure de pression (3) est placée à l'intérieur d'une veine (2) d'une combinaison de protection contre l'accélération.

3. Dispositif selon la revendication 1, **caractérisé en ce que** la cellule de mesure de pression (3) est placée à l'intérieur d'un récipient qui est raccordé au moyen d'un flexible à une veine (2) de la combinaison de protection contre l'accélération de façon à être en liaison par communication par liquide et pression avec cette veine (2).

4. Dispositif selon la revendication 2 ou 3, **caractérisé en ce que** la cellule de mesure de pression (3) se situe à hauteur du point le plus profond de la veine (2) de la combinaison de protection contre l'accélération.

5. Dispositif selon la revendication 2 ou 3, **caractérisé en ce que** la cellule de mesure de pression (3) est reliée au moyen d'un câble (5) à l'appareil d'évaluation (4), lequel câble (5) transmet à l'appareil d'évaluation (4) les valeurs captées par la cellule de mesure de pression (3).

6. Dispositif selon la revendication 2 ou 3, **caractérisé en ce qu'**un coupleur optique est prévu et raccordé à la cellule de mesure de pression (3), lequel transmet à l'appareil d'évaluation (4) les valeurs captées par la cellule de mesure de pression (3).

7. Dispositif selon la revendication 2 ou 3, **caractérisé en ce qu'**un appareil radio est prévu et raccordé à la cellule de mesure de pression (3), lequel transmet à l'appareil d'évaluation (4) les valeurs captées par la cellule de mesure de pression (3).

8. Dispositif selon la revendication 1, **caractérisé en ce qu'**un dispositif de visualisation (6) est prévu et raccordé à l'appareil d'évaluation (4).

9. Dispositif selon la revendication 1, **caractérisé en ce qu'**un dispositif à mémoire (7) est prévu et raccordé à l'appareil d'évaluation (4).

10. Dispositif selon la revendication 1, **caractérisé en ce qu'**on a prévu à la fois un dispositif de visualisation (6) et un dispositif à mémoire (7) et que les deux sont raccordés à l'appareil d'évaluation (4).
